# EUROPEAN PATENT APPLICATION

(11) **EP 4 151 152 A1**
(43) Date of publication of application: **22.03.2023**
(21) Application number: 21197725.1
(22) Date of filing: 20.09.2021
(51) Int. Cl.: A61B 5/16, A61B 5/00, A61B 5/055, G16H 20/70, A61B 5/291, A61B 5/369

(54) **ADAPTIVE TESTING FOR IMPULSE CONTROL**

(71) Applicant: Koninklijke Philips N.V., 5656 AG Eindhoven (NL)
(72) Inventor: VAN DOOREN, Marieke, Eindhoven (NL); HUIJBERS, Willem, Eindhoven (NL); VAN EE, Raymond, Eindhoven (NL); DENISSEN, Adrianus Johannes Maria, Eindhoven (NL); MENA BENITO, Maria Estrella, Eindhoven (NL); LAMERICHS, Rudolf Mathias Johannes Nicolaas, Eindhoven (NL)
(74) Representative: Philips Intellectual Property & Standards

(57) **Abstract**

A device for adapting testing parameters of a test, wherein the test comprises presenting congruent stimuli and incongruent stimuli to a subject. The device comprises a processor configured to obtain brain activity data corresponding to the brain activity of the subject responding to stimuli and adapt at least one testing parameter of the test based on the brain activity data.

## Description

### FIELD OF THE INVENTION

The invention relates to the field of tests consisting of the presentation of congruent and incongruent stimuli. In particular, the invention relates to the field of adapting at least one testing parameter of said tests.

### BACKGROUND OF THE INVENTION

Psychiatric disorders such as borderline personality disorder (BPD) or attention-deficit/hyperactivity disorder (ADHD) are characterized by impulsive behaviors. Several different psychological tests have been developed to quantify different components of impulse control (e.g. selective attention, response selection, motivational control, and behavioral inhibition). A conventional test is the Stroop test that measures response inhibition through incongruent word-color trials.

In clinical practice, in particular when working with children, it is conventional to start the Stroop test with two control tasks, a reading task where the subject has to read words of colors (Word task), and a Color task where the subject has to identify the color of bar charts. These two (Word and Color) tasks are relatively simple and can be used to check for reading disabilities and color blindness, but they can also serve the purpose of providing a baseline reaction time to identify the reading speed and the color naming speed and to identify a baseline error rate.

After providing a baseline, the actual Word-Color stimuli are presented where there are congruent stimuli and incongruent stimuli. In the congruent stimuli, the word of the color is the same as the color of the ink. Congruent stimuli are considered to be easy to respond to as there is no interference and no response inhibition is required. Hence, the reaction time and error rate are low.

The difficult stimuli to respond to are the incongruent stimuli where the color of the ink that should be identified differs from the meaning of the word. In this case, the reading of the word is the automatic response that should be suppressed (inhibited) in order to be able to name the color of the ink. When subjects are asked to name the color of the incongruent stimuli, the reaction time and error rate go up. The reaction time and error rate can be used as a diagnostic tool to situate the performance of a subject on the impulse control test within a distribution of normal, healthy, subjects against psychiatric subjects. If the maximal performance level of the subject falls in, for example, the 5% most extreme cases of the distribution, one can decide to further investigate whether to diagnose the subject with an impulse control disability.

Thus, an objective test like the Stroop test that measures response inhibition in a controlled way is a good solution to quantify the severity of the psychiatric impulse control behavior. The advantage of the test is that it enables the quantification of a specific component of impulse behavior. However, the test only provides a quantitative result if the subject responds within a "sensitive range" of the test. Specifically, the test fails to provide relevant quantitative results if the subject easily completes all trials (i.e. responding to the stimuli) rapidly (i.e. 100% success rate) or if the subject fails to complete any trials (i.e. 0% success rate). Ideally the test provides an outcome away from these 2 extremes (e.g. between 20-80% of wrong answers) which is one example of a sensitive range. Thus, there is a need to improve objective tests, like the Stroop test, which aim to quantify impulsive behavior.

### SUMMARY OF THE INVENTION

The invention is defined by the claims.

According to examples in accordance with an aspect of the invention, there is provided a device for adapting at least one testing parameter of a test, wherein the test comprises presenting congruent stimuli and incongruent stimuli to a subject, the device comprising a processor configured to:
obtain brain activity data corresponding to the brain activity of the subject responding to stimuli; and
adapt at least one testing parameter of the test based on the brain activity data.

Measuring brain activity can provide an indication of test difficulty. However the current state of the art does not use the activity of the brain in order to adapt the testing parameters (e.g. the difficulty) of the test.

The test may be a response inhibition test. The test may be a test for impulsive behavior of the subject. For example, the test may be a Stroop test. The test may consist of one or more tasks (e.g. control task and main task), wherein at least one task in the main task. Each task may consist of a plurality of trials, wherein each trial in the main task comprises the presentation of a congruent stimulus or an incongruent stimulus. The main task may be divided into blocks, wherein each block comprises a number of trials.

In psychology, the Stroop effect is the delay in reaction time between congruent and incongruent stimuli. The effect has been used to create psychological tests (e.g. the Stroop test) that are widely used in clinical practice and investigation.

The Stroop effect occurs when there is a mismatch between the name of a color (e.g., "blue", "green", or "red") and the color it is printed on (i.e., the word "red" printed in blue ink instead of red ink). When asked to name the color of the word, it takes longer, and is more prone to errors, when the color of the ink does not match the name of the color (this is an example of an incongruent trial). Congruent stimuli are those in which the ink color and the word refer to the same color (for example the word "pink" written in pink). Incongruent stimuli are those in which ink color and word differ.

More generally, a congruent stimulus is a stimulus which presents two or more pieces of information which are in agreement with each other. Similarly, an incongruent stimulus is a stimulus which presents two or more pieces of information which are not in agreement with each other.

However, the testing parameters (e.g. duration of time for which each stimulus is presented, number of colors, frequency of congruent/incongruent stimuli etc.) for these types of tests are usually generalized and are not personalized to the subject. Thus, some subjects may find a first set of parameters too difficult whilst other subjects may find the same first set of parameters too easy. If the test is too easy or too difficult, the responses of the subject to the stimuli may not be indicative of a particular behavior (e.g. impulsive behavior) of the subject.

The testing parameters may define how the stimuli are presented to the subject. The testing parameters may also be used to change the difficulty of the test. The testing parameters may determine a characteristic of the test. The testing parameters may include a parameter that is directly visible (e.g. the number of colors being used in a Stroop test), or it may be a parameter that is not directly visible (e.g. the number of congruent stimuli versus incongruent stimuli presented).

Thus, the inventors propose to obtain data corresponding to the brain activity of the subject whilst taking the test (i.e. whilst the stimuli is presented to the subject) and adapting the testing parameters according to the brain activity data. For example, if the test is found to be too easy due to the brain activity being below a lower threshold the testing parameters can be adapted to make the test more difficult. Similarly, if the (real time) acquisition of brain activity data falls above an upper threshold, then a testing parameter may be adapted (e.g. the test difficulty may be diminished). Preferable the brain activity data is obtained in real time (i.e. whilst the subject is responding to the test).

The brain activity data captures the intensity of brain activity for the subject.

The processor may be configured to adapt the testing parameters of the test based on the brain activity data showing an intensity of activation of the brain being below a lower threshold and/or the above an upper threshold.

For example, the brain activity data may contain the blood oxygen levels in the brain (e.g. obtained via fMRI ― functional magnetic resonance imaging), the intensity of electrical signals in the brain (e.g. obtained via EEG - electroencephalography) or the changes in hemoglobin in the brain (e.g. obtained via fNIRS - functional near-infrared spectroscopy).

The test may comprise a main task which involves presenting congruent and incongruent stimuli to the subject and tasking the subject with identifying a particular piece of information for each stimulus. The response to each stimulus is the identified information given by the subject.

The brain activity data may comprise activity data of, at least, the prefrontal cortex of the subject.

One aspect that is particularly relevant in psychiatry is the quantification of symptoms. In current practice, quantification is usually done through questionnaires which are not always replicable and indicative of the severity of the disease as there are different subjective motivations when filling in questionnaires (e.g. social desirability tendencies etc.). In particular, when the problem concerns impulsive behavior or response inhibition, this problem often manifests itself without the subject being aware of the impulsive behavior (i.e. the subject does not always realize the severity of the behavior).

The prefrontal cortex has been found to play an important role in response inhibition. When engaged in an impulse control situation, the oxygen that is required in the prefrontal cortex is elevated. For example, in fMRI, the measured blood oxygen level dependent (BOLD) response will increase in such a demanding behavioral response inhibition situation (i.e. when taking the test). Thus, measurements of brain activity of the prefrontal cortex may be used to enhance the measurement of impulsive behaviors when taking the test.

The testing parameters may comprise the frequency of congruent stimuli and incongruent stimuli presented to the subject and the processor may be configured to adapt the testing parameters by increasing the frequency of incongruent stimuli, and/or decreasing the frequency of congruent stimuli, if the brain activity data shows the intensity of activation of the brain being below a lower threshold, and decreasing the frequency of incongruent stimuli, and/or increasing the frequency of congruent stimuli, if the brain activity data shows the intensity of activation of the brain being above an upper threshold.

The testing parameters may comprise a set of colors, wherein each stimuli in the test can only correspond to a color in the set of colors, and the processor may be configured to adapt the testing parameters by increasing the number of colors in the set of colors if the brain activity data shows the intensity of activation of the brain being below a lower threshold, and decreasing the number of colors in the set of colors if the brain activity data shows the intensity of activation of the brain being above an upper threshold.

The testing parameters may comprise a time period corresponding to the presentation duration of time for which each stimuli is presented to the subject and the processor may be configured to adapt the testing parameters by decreasing the time period if the brain activity data shows the intensity of activation of the brain being below a lower threshold, and increasing the time period if the brain activity data shows the intensity of activation of the brain being above an upper threshold.

The test may further comprise control stimuli and the processor may be further configured to obtain baseline brain activity data corresponding to the brain activity of the subject when presented with the control stimuli and determine a lower threshold based on the baseline brain activity, wherein the processor is further configured to adapt the testing parameters based on the brain activity data showing the intensity of activation of the brain being below the lower threshold.

Typically, Stroop tests (or similar) are begun with control tasks (involving the presentation of control stimuli). The control tasks may include a reading task where the subject has to read words of colors and a color task where the subject simply has to identify the color of bar charts. These two control tasks can be used to check for reading disabilities and color blindness. Additionally, they can also be used to provide a baseline reaction time to identify the reading speed and the color naming speed of the subject. However, the control tasks make the test take longer.

The inventors propose to use the time being used for the control tasks to take a baseline activation level of the brain (and preferably of the prefrontal cortex). The control stimuli do not require any response inhibition of the brain (e.g. they may only require reading and color naming, without interference because of incongruency) and, thus, it is a good way to get a baseline for the activation intensity of the brain. The baseline can thus be used to determine a lower threshold for the brain activity.

The processor may be further configured to obtain historic responses to congruent stimuli and incongruent stimuli of the subject and the corresponding historic brain activity data and determine a lower threshold and/or an upper threshold based on the historic responses and the corresponding historic brain activity data, wherein the processor is further configured to adapt the testing parameters based on the brain activity data showing the intensity of activation of the brain being below the lower threshold and/or above the upper threshold.

For example, for a Stroop test, the first 10 words of the test could be used to adapt the testing parameters to an appropriate level of difficulty for the subject. Alternatively, the responses of a previous test (and corresponding brain activity data) may be used to adapt the testing parameters.

The test may consist of a number of blocks, each block consisting of a number of trials (i.e. presenting the stimuli). Thus, the historic brain activity data may consist of the brain activity data of the subject during previous (finished) blocks. Additionally, the thresholds (upper and lower) may be determined dynamically over time based on the brain activity of the subject during the last block. The thresholds (upper and lower) may be iteratively adapted based on changes in the historic brain activity data of the subject.

The intensity of activation of the brain may correspond to the activation of one or more regions of the brain (e.g. the prefrontal cortex) or of a brain network (e.g. the default mode network).

The invention also provides a system for adapting at least one testing parameter of a test, wherein the test comprises presenting congruent stimuli and incongruent stimuli to a subject, the system comprising:
a device for adapting at least one testing parameter of the test, as defined above;
a screen for presenting the stimuli to the subject; and
a brain activity measurement device configured to obtain the brain activity data of the subject.

The brain activity measurement device may be configured to perform fMRI, EEG and/or fNIRS. Preferably, the brain activity measurement device is an MRI device configured to perform fMRI.

The system may further comprise a response box for obtaining a response of the subject to the stimuli. Preferably, the response box comprises only two buttons (i.e. corresponding to yes/no responses). If the response box has a lot of buttons, the reaction time and error rate may increase due to the additional burden of selecting the correct finger to press the correct button. Thus, a response box with two buttons (yes/no) can be used with, for example, an adapted version of the Stroop test further explained below. Also preferably, the response box is compatible with an MRI device.

The invention also provides a method for adapting at least one testing parameter of a test, wherein the test comprises presenting congruent stimuli and incongruent stimuli to a subject, the method comprising:
obtaining brain activity data corresponding to the brain activity of the subject responding to stimuli; and
adapting at least one testing parameter of the test based on the brain activity data.

The brain activity data may comprise activity data of, at least, the prefrontal cortex of the subject.

The testing parameters may comprise the frequency of congruent stimuli and incongruent stimuli presented to the subject and wherein adapting the testing parameters may comprise increasing the frequency of incongruent stimuli, and/or decreasing the frequency of congruent stimuli, if the brain activity data shows the intensity of activation of the brain being below a lower threshold and decreasing the frequency of incongruent stimuli, and/or increasing the frequency of congruent stimuli, if the brain activity data shows the intensity of activation of the brain being above an upper threshold.

The test may further comprise control stimuli and the method may further comprise obtaining baseline brain activity data corresponding to the brain activity of the subject when presented with the control stimuli and determining a lower threshold based on the baseline brain activity, wherein adapting the testing parameters is further based on the brain activity data showing the intensity of activation of the brain being below the lower threshold.

The method may further comprise obtaining historic responses to congruent stimuli and incongruent stimuli of the subject and the corresponding historic brain activity data and determining a lower threshold and/or an upper threshold based on the historic responses and the corresponding historic brain activity data, wherein adapting the testing parameters is further based on the brain activity data showing the intensity of activation of the brain being below the lower threshold and/or above the upper threshold.

The invention also provides a computer program product comprising computer program code which, when executed on a computing device having a processing system, cause the processing system to perform all of the steps of the afore-mentioned method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a method for adapting the testing parameters;
Fig. 2 shows four example stimuli of a Stroop test;
Fig. 3 shows an adapted version of the Stroop test; and
Fig. 4 shows a system with a processor and an MRI scanner.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a device for adapting testing parameters of a test, wherein the test comprises presenting congruent stimuli and incongruent stimuli to a subject. The device comprises a processor configured to obtain brain activity data corresponding to the brain activity of the subject responding to stimuli and adapt at least one testing parameter of the test based on the brain activity data.

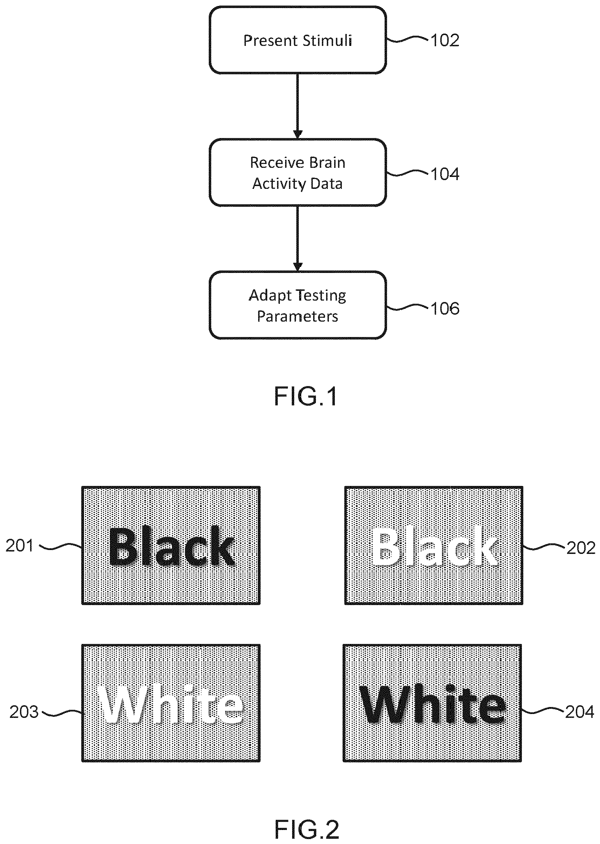

Fig. 1 shows a method for adapting the testing parameters of a test that involves presenting congruent stimuli and incongruent stimuli to a subject. The test difficulty is improved by adapting the testing parameters based on the brain activity data of the subject. When the stimuli (congruent and incongruent) are presented, in step 102, to the subject, the subject is tasked with responding to each stimuli (e.g. naming the color of the ink of the presented word). Brain activity data, in step 104, is obtained whilst the subject is responding to the stimuli. The brain activity data is then used, in step 106, to adapt one or more testing parameters of the test in order to adapt the difficulty of the test.

It is proposed to adapt the difficulty of the test based on the prefrontal cortex activity to ensure the test is operating in a sensitive range. It is well known from literature that the prefrontal cortex is correlated with impulse control difficulties. Therefore, it is advantageous to use the activation level of the prefrontal cortex when adapting the testing parameters of, for example, a Stroop test.

A functional brain activation monitor (e.g. fMRI, EEG, fNIRS etc.) may be used to measure the brain activity data. For example, the prefrontal cortex activity intensity, the activity of other brain areas, the activity of the whole brain areas and/or the activity of brain networks (e.g. the default mode network) may be measured.

Real time fMRI provides functional information for each time point. Functional variation in other areas of the brain (other than the prefrontal cortex) may also be detected. These variations can be an additional measurement of the brain activity data. Similarly, other areas of the brain may be measured with EEG or fNIRS.

An impulse behavior test (e.g. a Stroop test) may be set up with a monitor to project word-color stimuli and a response box may be used to register the responses of the subjects taking the test.

A processor is configured to dynamically adjust the testing parameters of the test (e.g. frequency of incongruent stimuli, presentation duration, number of colors etc.) based on the measured brain activity data in order to ensure the test is operating in its sensitive range (e.g. 20-80% of maximum brain activity intensity). Specifically, if the brain activity intensity is below a lower threshold, then the difficulty of the test is increased by adjusting the parameters accordingly. Similarly, if the brain activity intensity is above an upper threshold, then the difficulty of the test is decreased by adjusting testing parameters accordingly.

The identification of illness related impulse control problematic behavior may rely on the determination of the ceiling performance of a subject. The ceiling performance can be determined by analyzing the reaction time and the number of incorrect responses (i.e. errors) in the test. The performance should be situated in a normal distribution of a wide generous population of normal, healthy, subjects and subject suffering from impulse control problems like psychiatric subjects suffering from ADHD or BPD.

Fig. 2 shows four example stimuli of a Stroop test. Stimuli 201 and 203 are congruent stimuli as the color of the ink of the word matches the meaning of the word. Stimuli 202 and 204 are incongruent stimuli as the color of the ink of the word does not match the meaning of the words.

The following example embodiments are related to the testing parameters of the Stroop color and word test. However, it will be understood by a person skilled in the art that other similar tests (involving congruent and incongruent stimuli) may be used, wherein the testing parameters will relate to how the stimuli are presented to the subject for each type of test. Examples of different tests include the warped words Stroop test, the emotional Stroop test, the spatial Stroop test, the numerical Stroop test and the reverse Stroop test. The use of "Stroop" in all of the afore-mentioned tests is merely naming convention for these types of tests and other types tests without the "Stroop" naming convention may be envisioned by a person skilled in the art.

In a first embodiment, the frequency of congruent and incongruent stimuli are adapted based on real-time brain activation data of prefrontal cortex. In other words, the ratio of congruent to incongruent stimuli presented to the subject is adapted by changing the frequency of one or more of the congruent and incongruent stimuli. The Stroop color and word test is given to a subject inside an MRI scanner with an MR compatible response button box to measure the reaction time upon presentation of word-color stimuli on a monitor inside the bore (or outside the bore), where the subject inside the bore of the MRI scanner can see the content of the monitor via a mirror projection (i.e. a mirror placed on the head coil).

The brain activation intensity in the prefrontal cortex is measured in real time and is analyzed to determine whether the activation intensity falls below a lower threshold, thus giving an indication of the difficulty of the test. If it is determined that the difficulty level of the Stroop test is too simple (i.e. brain activation data below lower threshold intensity level), then the number of incongruent word-color stimuli may be increased. Gradually increasing the frequency of incongruent word-color stimuli (in real time or with a short delay per block of stimuli) may lead to an increased reaction time and an increased amount of wrong responses as the automatic reading of the meaning of the word needs to be suppressed whilst the color of the word needs to be detected as fast as possible.

The difficulty level of the Stroop color and word test is personalized for each subject based on the brain activity data and, optionally, dynamic thresholds (i.e. changing over time) which quantify when the subject may be finding the test too easy or difficult. Ideally, the thresholds are dynamic over time as this may prevent slight but significant learning effects where the subject learns how to perform better on the Stroop test.

The reaction time (i.e. the time between stimuli being presented and a response) and the number of incorrect responses may also provide good insight into the skills of response inhibition/impulse control of the subject. If the reaction time of incongruent stimuli increases (and/or if the number of errors of incorrect answers increases), this may give a good indication that the subject has crossed their (personal) ceiling of adequate behavioral impulse control.

The estimation of adequate versus inadequate behavioral impulse control may also come from a comparison of the subject's performance on the test, where the number of errors and the reaction times are indicative (e.g. through thresholds) of the performance, compared to the values of a group of healthy control subjects. The determination of illness may be aided by situating of the subject's performance within a distribution of data that comes from a generous population of healthy and, for example, of psychiatric ADHD subjects.

In summary, the prefrontal cortex activity may be measured in real time and used to adjust the frequency of incongruent stimuli (and/or congruent stimuli) of the Stroop test. It the brain activity intensity is below a certain threshold, then the frequency may be increased. Gradually increasing the frequency of incongruent stimuli may lead to more incorrect answers and a longer reaction time. If the number of incorrect answers and reaction times reach a (personal) ceiling, then this value of the ceiling can be situated in a normal distribution of a wide and generous population of healthy and, for example, of psychiatric ADHD subjects. If the ceiling value of the Stroop test falls within the 5% most extreme observations, then it may be very likely that the subject suffers from a psychiatric disease that is characterized by compromised impulse control. If the ceiling value of the Stroop test falls within the 50% average observations, then it is very unlikely that the subject suffers from a psychiatric disease that is characterized by compromised response inhibition.

In a second embodiment, the number of colors in the Stroop color and word test are adapted based on real-time brain activity data of prefrontal cortex. The difficulty level of the Stroop test can also be adapted by increasing the number of colors being used in the stimuli.

In a simple version of the Stroop test, only four colors are used. Using only four colors is easier for the subject as they only need to suppress three incorrect responses when presented with an incongruent stimulus. In a more difficult version of the Stroop test, eight colors can be used. This makes the Stroop test more difficult as the subject needs to suppress seven incorrect answers in the case of an incongruent stimulus.

The adaptation of the number of colors being used in the Stroop test may be based on the real-time measured brain activity data of the prefrontal cortex. If it is detected that the prefrontal cortex activity intensity is below a lower threshold, then the number of colors being used in the Stroop test may be increased.

In the second embodiment where the number of colors in the Stroop test is adapted, it may be necessary to consider adapting the response box where the subject provides his responses to the test. Several different implementations of a response box exist for the Stroop test (e.g. response box with four buttons, with six buttons etc.). However, it is important to make the appropriate choice of response box for a good implementation. In the case where it is decided to make use of eight different colors, it is not practical to use a response box with eight buttons (i.e. one for each color). This may lead to interference in the Stroop effect as, if a person needs to press one of eight buttons with one hand, the brain activity becomes a measurement of motor selection (i.e. with which finger does the subject need to use to press the correct button) instead of a measurement of impulse control. Additionally, due to the interference of the motor selection, the reaction time and the number of incorrect answers would artificially increase.

Thus, the inventors propose a new response box implementation with two buttons. When using the new response box, the original version of the Stroop test may be slightly adapted, whereby now there are two types of stimuli which follow up on each other.

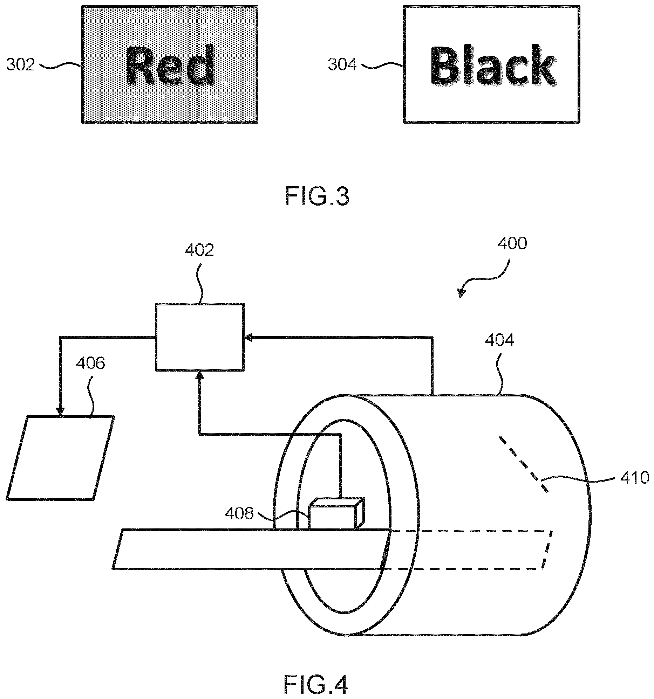

Fig. 3 shows an adapted version of the Stroop test. The first type of stimuli 302 corresponds to the regular word-color stimuli (i.e. congruent or incongruent), and the second type of stimuli 304 corresponds to a descriptive word (e.g. in black or white). Now, the task of the subject is to verify, with a yes or no response (i.e. using one of the two buttons of the adapted response box), whether the meaning of the second type of stimulus 304 corresponds with the color of the word-color stimulus.

In a third embodiment, the speed at which stimuli are presented to the subject is adapted based on real-time brain activity data of the prefrontal cortex. In other words, the time period/duration of time for which each stimulus is presented to the subject is adapted. The speed at which each stimulus is presented to the subject also influences the difficulty of the Stroop test. The duration of each stimulus (i.e. for how long each stimulus is presented to the subject) can be adapted based on the prefrontal brain activation level (and its corresponding thresholds).

The lower threshold for brain activity data can be determined using control tasks for the Stroop test. Before presenting the main task of the test (i.e. presenting the congruent and incongruent stimuli), the subject may undergo, for example, two control tasks (word reading task and color naming tasks). This is particularly relevant when testing children with, for example, reading disabilities or in subjects with color blindness. In clinical practice, these two control tasks usually end up taking extra time to establish results.

However, it is proposed to use the time being used to perform the two control tasks to obtain a baseline activation level of the brain (and in particular of the prefrontal cortex). The two control tasks do not require any response inhibition instead merely requiring reading and color naming stimuli without any stimuli which require response inhibition. Thus, the control tasks (i.e. presenting control stimuli) enable the user to obtain a baseline activation level for the subject. The baseline activation level can be further used to determine a lower threshold for brain activity intensity.

An upper threshold may be determined by using previous tests performed by the subject. For example, it is proposed to use a previous test where the subject has "failed" to complete the test (e.g. <20% correct responses) to establish the upper threshold for the brain activity intensity.

It is also proposed to adjust the test difficulty based on the level of brain activity during the initial part of the test in order to ensure that the majority of the test is completed in the sensitive range of brain activity (i.e. between the lower threshold and upper threshold of brain activity intensity). For example, for the Stroop test, the first ten word-color stimuli may be used to calibrate the test for the following 100 stimuli.

Once an appropriate level of difficulty has been established to keep the brain activity intensity in the sensitive range, the responses of the subject to the test can be compared with results of earlier tests corresponding to the same level of difficulty. It may be advantageous to use discrete levels of difficulty (wherein each level of difficulty is defined by the testing parameters). For example, one of five levels of difficulty can be selected depending on the level of prefrontal cortex activity.

Alternatively, relative scores may be established between tests at different difficulty levels by applying a calibration factor (e.g. obtained from population data of results of subjects carrying out all the different difficulty levels of the test).

Fig. 4 shows a system with a processor 402 and an MRI scanner 404. The MRI scanner 404 is used to obtain brain activity data of a subject and send it to the processor 402. The processor 402 may also be used to display the test on a screen 406. Alternatively, a different processor may be used to display the test on the screen 406. A response box 408 is used to receive the responses to the test made by the subject. In some cases, a mirror 410 may be used to enable the subject to see the screen 406 from inside the MRI scanner 404. Alternatively, the screen 406 may be placed inside the MRI scanner 404 and there is no need for the mirror 410.

The skilled person would be readily capable of developing a processor for carrying out any herein described method. Thus, each step of a flow chart may represent a different action performed by a processor, and may be performed by a respective module of the processor.

As discussed above, the device makes use of processor to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A device for adapting at least one testing parameter of a test, wherein the test comprises presenting (102) congruent stimuli (201, 203) and incongruent stimuli (202, 204) to a subject, the device comprising a processor (402) configured to:
obtain (104) brain activity data corresponding to the brain activity of the subject responding to stimuli; and
adapt (106) at least one testing parameter of the test based on the brain activity data.

2. The device of claim 1, wherein the brain activity data comprises activity data of, at least, the prefrontal cortex of the subject.

3. The device of any one of claims 1 or 2, wherein the testing parameters comprise the frequency of congruent stimuli (201, 203) and incongruent stimuli (202, 204) presented to the subject and wherein the processor (402) is configured to adapt (106) the testing parameters by:
increasing the frequency of incongruent stimuli (202, 204), and/or decreasing the frequency of congruent stimuli (201, 203), if the brain activity data shows the intensity of activation of the brain being below a lower threshold; and
decreasing the frequency of incongruent stimuli (202, 204), and/or increasing the frequency of congruent stimuli (201, 203), if the brain activity data shows the intensity of activation of the brain being above an upper threshold.

4. The device of any one of claims 1 to 3, wherein the testing parameters comprise a set of colors, wherein each stimuli in the test can only correspond to a color in the set of colors and wherein the processor (402) is configured to adapt (106) the testing parameters by:
increasing the number of colors in the set of colors if the brain activity data shows the intensity of activation of the brain being below a lower threshold; and
decreasing the number of colors in the set of colors if the brain activity data shows the intensity of activation of the brain being above an upper threshold.

5. The device of any one of claims 1 to 4, wherein the testing parameters comprise a time period corresponding to the duration of time for which each stimuli is presented (102) to the subject and wherein the processor (402) is configured to adapt (106) the testing parameters by:
decreasing the time period if the brain activity data shows the intensity of activation of the brain being below a lower threshold; and
increasing the time period if the brain activity data shows the intensity of activation of the brain being above an upper threshold.

6. The device of any one of claims 1 to 5, wherein the test further comprises control stimuli and wherein the processor (402) is further configured to:
obtain baseline brain activity data corresponding to the brain activity of the subject when presented with the control stimuli; and
determine a lower threshold based on the baseline brain activity data, wherein the processor (402) is further configured to adapt (106) the testing parameters based on the brain activity data showing the intensity of activation of the brain being below the lower threshold.

7. The device of any one of claims 1 to 6, wherein the processor (402) is further configured to:
obtain historic responses to congruent stimuli (201, 203) and incongruent stimuli (202, 204) of the subject and the corresponding historic brain activity data; and
determine a lower threshold and/or an upper threshold based on the historic responses and the corresponding historic brain activity data, wherein the processor (402) is further configured to adapt (106) the testing parameters based on the brain activity data showing the intensity of activation of the brain being below the lower threshold and/or above the upper threshold.

8. A system (400) for adapting at least one testing parameter of a test, wherein the test comprises presenting (102) congruent stimuli (201, 203) and incongruent stimuli (201, 203) to a subject, the system (400) comprising:
a device according to any one of claims 1 to 7;
a screen (406) for presenting (102) the stimuli to the subject; and
a brain activity measurement device (404) configured to obtain (104) the brain activity data of the subject.

9. The system of claim 8, wherein the system (400) further comprises a response box (408) for obtaining a response of the subject to the stimuli.

10. A method for adapting at least one testing parameter of a test, wherein the test comprises presenting (102) congruent stimuli (201, 203) and incongruent stimuli (202, 204) to a subject, the method comprising:
obtaining (104) brain activity data corresponding to the brain activity of the subject responding to stimuli; and
adapting (106) at least one testing parameter of the test based on the brain activity data.

11. The method of claim 10, wherein the brain activity data comprises activity data of, at least, the prefrontal cortex of the subject.

12. The method of any one of claims 10 or 11, wherein the testing parameters comprise the frequency of congruent stimuli (201, 203) and incongruent stimuli (202, 204) presented to the subject and wherein adapting (106) the testing parameters comprises:
increasing the frequency of incongruent stimuli (202, 204), and/or decreasing the frequency of congruent stimuli (201, 203), if the brain activity data shows the intensity of activation of the brain being below a lower threshold; and
decreasing the frequency of incongruent stimuli (202, 204), and/or increasing the frequency of congruent stimuli (201, 203), if the brain activity data shows the intensity of activation of the brain being above an upper threshold.

13. The method of any one of claims 10 to 12, wherein the test further comprises control stimuli and wherein the method further comprises:
obtaining baseline brain activity data corresponding to the brain activity of the subject when presented with the control stimuli; and
determining a lower threshold based on the baseline brain activity data, wherein adapting (106) the testing parameters is further based on the brain activity data showing the intensity of activation of the brain being below the lower threshold.

14. The method of any one of claims 10 to 13, further comprising:
obtaining historic responses to congruent stimuli (201, 203) and incongruent stimuli (202, 204) of the subject and the corresponding historic brain activity data; and
determining a lower threshold and/or an upper threshold based on the historic responses and the corresponding historic brain activity data, wherein adapting (106) the testing parameters is further based on the brain activity data showing the intensity of activation of the brain being below the lower threshold and/or above the upper threshold.

15. A computer program product comprising computer program code which, when executed on a computing device having a processing system, cause the processing system to perform all of the steps of the method according to any of claims 10 to 14.
